## Europäisches Patentamt
## European Patent Office
## Office européen des brevets

(19)

(11) Publication number : **0 424 391 B1**

(12) # EUROPEAN PATENT SPECIFICATION

(45) Date of publication of patent specification :
**09.12.92 Bulletin 92/50**

(51) Int. Cl.$^5$ : **A61K 6/08**

(21) Application number : **89904280.8**

(22) Date of filing : **17.03.89**

(86) International application number :
**PCT/SE89/00142**

(87) International publication number :
**WO 89/09045 05.10.89 Gazette 89/24**

(54) **COMPOSITE MATERIAL KIT FOR DENTAL FILLINGS AND METHOD FOR DETERMININING THE COLOUR OF A DENTAL FILLING.**

(30) Priority : **25.03.88 SE 8801100**

(43) Date of publication of application :
**02.05.91 Bulletin 91/18**

(45) Publication of the grant of the patent :
**09.12.92 Bulletin 92/50**

(84) Designated Contracting States :
**AT BE CH DE FR GB IT LI LU NL**

(56) References cited :
**US-A- 4 512 743**
**US-A- 4 608 015**
**US-A- 4 715 813**

(73) Proprietor : **NORDISKA DENTAL AB**
**Box 911**
**S-251 09 Helsingborg (SE)**

(72) Inventor : **LEMPERT, John**
**Föreningsgatan 15**
**S-252 38 Helsingborg (SE)**

(74) Representative : **Berglund, Gustav Arthur et al**
**AWAPATENT AB Box 5117**
**S-200 71 Malmö (SE)**

EP 0 424 391 B1

## Description

The present invention relates to composite materials for dental fillings and methods for determining the colour of a dental filling in order to match the colour of the filling to that of the adjoining tooth substance.

It is known to use for dental fillings, primarily front tooth fillings, different types of composite material usually consisting of an organic or inorganic filler and a plastics base which is polymerisable, for example by means of light.

To make a filling of a composite material fully match the surrounding teeth in respect of colour, the dentist must make a difficult selection of a suitable colour and a subsequent uncertain practical matching of the colour of the composite material on the basis of the colour selected.

The suitable colour has frequently been established by comparing the colour of the patient's teeth with a large range of colour samples. Once the colour sample which best matches with the colour of the patient's teeth has been selected, the composite material to be utilised has been applied.

It has been found that matching the colour of the composite material is a problem which most dentists have in common. For a successful result, the dentist must have, inter alia, a sound knowledge of chromatics.

It is the object of this invention to simplify matching of the colour of a dental filling with that of the adjoining tooth substance.

This object is achieved by means of a composite material kit for dental fillings. According to the invention, the kit is characterised by a base composite material, the three-dimensional colour value of which, expressed as hue, chroma and blackness/whiteness, so corresponds to that of an average selection of natural teeth that the visual impression of tooth and filling will be the same, and at least three further composite materials, the three-dimensional colour values of which deviate in different directions from the colour value of the base composite material in such a way that adequate coverage is obtained of the colour value dispersion of natural teeth.

Proceeding from the known colour value dispersion of natural tooth material (see for example the series of articles published by Robert C. Sproull in J. Prosthet. Dent. Vol. 29, No. 4, April 1973, pp. 416-424; Vol. 29, No. 5, May 1973, pp.556-566; and Vol. 31, No. 2, Feb. 1974, pp. 146-154), it could be shown by experiments conducted in connection with the present invention that a division of the problem situations into a number of principal areas gave the solution according to the invention. To achieve adequate coverage, four principal areas should suitably be defined which are readily discernible to the dentist. Admittedly, it is possible to restrict the number of principal areas to three, or to increase the number of principal areas to five or more, but in the first instance the coverage of the colour value dispersion of the natural tooth material will not be as satisfactory, and more than five principal areas make the colour selection too difficult for the dentist.

A first one of the said principal areas has been defined as comprising whiter tooth material, and a second one as comprising blacker tooth material than the average tooth material. In the composite material kit according to the invention, two further composite materials have therefore been incorporated, of which a first one is whiter and a second one is blacker than the base composite material.

For the case with but three principal areas, it would be possible to include in the composite material kit according to the invention a third further composite material which is more coloured and blacker than the base composite material.

In the preferred embodiment of the invention, however, there is used, besides the first-mentioned two principal areas, also a third principal area comprising more yellowish tooth materials than the average tooth material, and a fourth principal area comprising more coloured and blacker tooth material than the average tooth material. In the composite material kit according to the invention, a third further composite material which is more yellowish than the base composite material, and a fourth further composite material which is more coloured and blacker than the base composite material, have been incorporated.

All of the said further composite materials included in the kit preferably have one and the same opacity, a suitable value of which is approximately 60%.

In the most preferred embodiment of the composite material kit according to the invention, the base composition material is included in two variants in respect of opacity, one variant having the same opacity as the further composite materials, and the second variant having a lower opacity, for example 40%.

According to the invention, the last-mentioned variant of the base composite material can be utilised for varying the degree of deviation from the colour of the base composite material of the finished tooth filling, more particularly by providing the variant having the lower opacity in the form of a layer on top of a layer of some of the further composite materials. By varying the relative thicknesses of the two layers, the degree of deviation from the colour of the base composite material towards the selected further composite material can be readily controlled.

As has been mentioned above, a colour can be characterised as a three-dimensional colour value in which the three dimensions or parameters represent hue, chroma and blackness/whiteness. This characterisation is always possible, although it is not the only one possible. A known system of this characterisation is the so-called natural colour system usually termed the NCS or NC system which indicates by a sequence of digits and letters first the degree of blackness by a two-digit percentage, then the degree of chroma by a two-digit percentage, and finally the hue by a first primary colour indication followed by a two-digit percentage for a subsequent second primary colour indication, for example 1020-Y30R where Y represents yellow and R represents red.

In an especially preferred embodiment of the composite material kit according to the invention, the base composite material has approximately the colour value in the NC system which is designated 1525-Y30R, and the further composite materials have approximately the colour values which in the NC system are designated 1010-Y30R (whiter), 3020-Y30R (blacker), 1030-Y10R (more yellowish) and 3030-Y30R (more coloured and blacker).

In a method for determining the colour of a dental filling of composite materials for matching the colour of the filling to that of the adjoining tooth substance, the above-mentioned object of the invention is achieved in that the colour is determined by selecting one of at least four and preferably five composite materials, of which a base composite material has a three-dimensional colour value, expressed as hue, chroma and blackness/whiteness, which so corresponds to that of an average selection of natural teeth that the visual impression of tooth and filling will be the same, and the other composite materials have colour values deviating from the colour value of the base composite material towards whiter, blacker and more coloured with higher blackness or, in the preferred case, more yellowish and more coloured with higher blackness, respectively.

Furthermore, the colour can be matched by selecting the thickness of an outer layer of the filling, the composite material of the inner layer being selected among one of the other composite materials, and the outer layer being formed of the base composite material which, in this case, has a lower opacity than the remaining composite materials, and/or a transparent composite material.

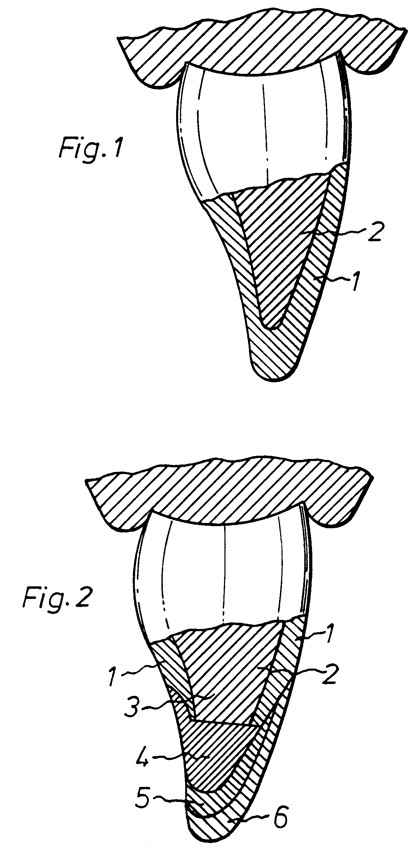

A practical utilisation of the composite material kit and the method according to the invention will now be described below, reference being had to the accompanying drawing i which Fig. 1 is a partial cross-sectional view of a front tooth without filling, and Fig. 2 is a partial cross-sectional view having a filling according to the invention.

Teeth generally have an outer layer of enamel 1 over the principal tooth material 2, called dentine. The enamel is more translucent than the dentine, and in the case of the front tooth the thickness of the enamel layer usually decreases in the cervical direction on the tooth, as shown in Fig. 1. In view hereof, a normal front tooth is relatively transparent incisally.

Before a filling is built up, the cavity at issue is enlarged, or those parts of the tooth that are to be replaced, are removed. Fig. 2 illustrates a situation in which a large part of a front tooth must be replaced incisally. A post 3 has been formed, and about this post the filling is to be built up.

In a first step, the surface of the enamel is etched, whereupon a bonding material that can be bonded micro-mechanically to the etched enamel 1, is applied. If the post 3 is discoloured, it is possible, before application of the composite material according to the invention, to apply an essentially entirely opaque and covering primer to mask the discoloration, whereupon the composite material is applied either by a one-step procedure or by a two-step procedure.

The one-step procedure is utilised in the event that the colour of the tooth and the surrounding teeth matches that of the base composite material or that of one of the said further composite materials.

The two-step procedure which is illustrated in Fig. 2, is utilised in the event that the colour deviation between the tooth and the surrounding teeth from the base composite material is not as pronounced as the colour deviations of the further composite materials. In this case, a layer 4 of the further composite material at issue is first applied, followed by a layer 5 of the base composite material in the variant having the lower opacity. The result of this sequence of the layers is a correct build-up of the tooth in respect of colour, with a more opaque, more strongly coloured inner part and a more translucent outer part, such that the repaired tooth will have a natural lustre. To further increase the lustre, it is possible, both in the one-step and in the two-step procedure, to use a transparent material for forming a thin surface layer 6 on the tooth, and this transparent material may be highly translucent.

The above-mentioned examples of colour values for the composite materials according to the invention have been determined at a given thickness (~1.5 mm) of the solidified composite material and with a given background (a grey hue corresponding to the blackness 1500 of the NC system).

Summing up, it will be appreciated that the composite material kit according to the invention greatly facilitates the dentist's work in that it provides, in a simple and convenient manner, a starting point for the colour of a filling, which the dentist just as simply and conveniently can modify with regard to other objective and subjective factors affecting the visual impression of the repair, such as lighting, surface area, viewing angle, surrounding colours, and background.

For a large proportion of patients, perhaps fifty percent or more, the base composite material is sufficient because its colour value has been selected to lie in a statistical centre of gravity of the colour value dispersion of natural teeth.

For example, two or more opaque and covering primers may be used, whereby the colour deviation relative to the colour value of the base composite material can be increased.

## Claims

1. Composite material kit for dental fillings, **characterised** by a base composite material, the three-dimensional colour value of which, expressed as hue, chroma and blackness/whiteness, so corresponds to that of an average selection of natural teeth that the visual impression of tooth and filling will be the same, and at least three further composite materials, the three-dimensional colour values of which deviate in different directions from the colour value of the base composite material in such a way that adequate coverage is obtained of the colour value dispersion of natural teeth.

2. Composite material kit as claimed in claim 1, **characterised** in that it contains the base composite material in two variants having the same colour value but different opacity, preferably about 40% and 60% opacity, of which variants the one having the lower opacity is adapted to be applied in the form of a layer on top of any of said further composite materials.

3. Composite material kit as claimed in claim 2, **characterised** in that said further composite materials have essentially the same opacity as the more opaque variant of the base composite material.

4. Composite material kit as claimed in any one of claims 1-3, **characterised** in that a first one of said further composite materials, expressed in the NC system, is whiter and a second one blacker than said base composite material.

5. Composite material kit as claimed in any one of claims 1-4, **characterised** in that a third of said further composite materials, expressed in the NC system, is more coloured and blacker than the base composite material.

6. Composite material kit as claimed in any one of claims 1-**4, characterised** in that a third one of said further composite materials, expressed in the NC system, is more yellowish, and a fourth one more coloured and blacker than the base composite material.

7. Composite material kit as claimed in claim 6, **characterised** in that the base composite material has the colour value which, in the NC system, is designated 1525-Y30R, and said further composite materials the colour values which, in the NC system, are designated 1010-Y30R (whiter), 3020-Y30R (blacker), 1030-Y10R (more yellowish) and 3030-Y30R (more coloured and blacker).

8. Composite material kit as claimed in any one of claims 1-7, **characterised** in that it also comprises at least one opaque material for covering discolourations underneath the composite material filling, and a translucent material for forming a surface layer.

9. Method for determining the colour of a dental filling of composite material for matching the colour of the filling to that of the adjoining tooth substance, **characterised** in that the colour is determined by selecting one of at least four and preferably five composite materials, of which a base composite material has a three-dimensional colour value, expressed as hue, chroma and blackness/whiteness, which so corresponds to that of an average selection of natural teeth that the visual impression of tooth and filling will be the same, and the other composite materials have colour values deviating from the colour value of the base composite material towards whiter, blacker and more coloured with higher blackness or, in the preferred case, more yellowish and more coloured with higher blackness, respectively.

10. Method as claimed in claim in claim 9, **characterised** in that the colour is matched by selecting the thickness of an outer layer of the filling, the composite material of the inner layer being selected among one of the other composite materials, and the outer layer being formed of the base composite material which, in this case, has a lower opacity than the remaining composite materials, and/or a transparent composite material.

## Patentansprüche

1. Compositwerkstoffsatz für Zahnfüllungen, **gekennzeichnet** durch einen Grundcompositwerkstoff, dessen räumlicher Farbwert, ausgedrückt als Farbton, Chroma und Schwärze/Weisse, in einer solchen Weise dem Farbwert einer Durchschnittsauswahl natürlicher Zähne entspricht, dass der optische Eindruck von Zahn und Füllung derselbe sein wird, und durch zumindest drei weitere Compositwerkstoffe, deren räumliche Farbwerte in verschiedenen Richtungen in einer solchen Weise vom Farbwert des Grundcompositwerkstoffs abweichen, dass eine gute Deckung der Farbwertstreuung natürlicher Zähne erzielt wird.

2. Compositwerkstoffsatz nach Anspruch 1, dadurch **gekennzeichnet,** dass er den Grundcompositwerkstoff in zwei Varianten enthält, welche denselben Farbwert, jedoch unterschiedliche Opazität, vorzugsweise etwa 40% und 60% Opazität, aufweisen, und von welchen die Variante der niedrigeren Opazität in Form einer Schicht dem einen der genannten, weiteren Compositwerkstoffe aufgetragen ist.

3. Compositwerkstoffsatz nach Anspruch 2, dadurch **gekennzeichnet**, dass die genannten, weiteren Compositwerkstoffe im wesentlichen dieselbe Opazität haben wie die opakere Variante des Grundcompositwerkstoffs.

4. Compositwerkstoffsatz nach einem der Ansprüche 1-3, dadurch **gekennzeichnet,** dass ein erster der genannten, weiteren Compositwerkstoffe, ausgedrückt im NC-System, weisser ist, und ein zweiter schwärzer ist als der genannte Grundcompositwerkstoff.

5. Compositwerkstoffsatz nach einem der Ansprüche 1-4, dadurch **gekennzeichnet,** dass ein dritter der genannten, weiteren Compositwerkstoffe, ausgedrückt im NC-System, mehr gefärbt und schwärzer ist als der Grundcompositwerkstoff.

6. Compositwerkstoffsatz nach einem der Ansprüche 1-4, dadurch **gekennzeichnet**, dass ein dritter der genannten, weiteren Compositwerkstoffe, ausgedrückt im NC-System, gelblicher ist, und ein vierter mehr gefärbt und schwärzer ist als der Grundcompositwerkstoff.

7. Compositwerkstoffsatz nach Anspruch 6, dadurch **gekennzeichnet**, dass der Farbwert des Grundcompositwerkstoffs im NC-System die Bezeichnung 1525-Y30R trägt, während die Farbwerte der genannten, weiteren Compositwerkstoffe im NC-System die Bezeichnungen 1010-Y30R (weisser), 3020-Y30R (schwärzer), 1030-Y10R (gelblicher) und 3030-Y30R (mehr gefärbt und schwärzer) tragen.

8. Compositwerkstoffsatz nach einem der Ansprüche 1-7, dadurch **gekennzeichnet**, dass er ferner zumindest einen opaken Werkstoff zur Abdeckung von Verfärbungen unter der Compositwerkstofffüllung und einen lichtdurchlässigen Werkstoff zur Bildung einer Oberflächenschicht aufweist.

9. Verfahren zur Bestimmung der Farbe einer Zahnfüllung aus Compositwerkstoff, um die Farbe der Füllung der Farbe des angrenzenden Zahnstoffs anzupassen, dadurch **gekennzeichnet**, dass die Farbe durch Wählen von einem von zumindest vier und vorzugsweise fünf Compositwerkstoffen bestimmt wird, von denen ein Grundcompositwerkstoff einen räumlichen Farbwert, ausgedrückt als Farbton, Chroma und Schwärze/Weisse, aufweist, der in einer solchen Weise dem Farbwert einer Durchschnittsauswahl natürlicher Zähne entspricht, dass der optische Eindruck von Zahn und Füllung derselbe sein wird, und die anderen Compositwerkstoffe Farbwerte aufweisen, die von dem Farbwert des Grundcompositwerkstoffs abweichen, und zwar in Richtung weisser, schwärzer und mehr gefärbt mit höherer Schwärze oder, im bevorzugten Falle, gelblicher bzw. mehr gefärbt mit höherer Schwärze.

10. Verfahren nach Anspruch 9, dadurch **gekennzeichnet**, dass die Farbe durch Wählen der Dicke einer äusseren Schicht der Füllung angepasst wird, wobei der Compositwerkstoff der inneren Schicht unter den anderen Compositwerkstoffen gewählt wird, und die äussere Schicht aus dem Grundcompositwerkstoff, der in diesem Fall eine niedrigere Opazität hat als die übrigen Compositwerkstoffe, und/oder einem durchsichtigen Compositwerkstoff gebildet wird.

## Revendications

1. Ensemble de matériaux composites pour obturations dentaires, caractérisé par un matériau composite de base dont la valeur chromatique tridimensionnelle, exprimée en teinte, degré de teinte et noirceur/blancheur, correspond à celle d'une sélection moyenne des dents naturelles de sorte que l'impression visuelle de la dent et de l'obturation sera la même, et par au moins trois matériaux composites supplémentaires dont les valeurs chromatiques tridimensionnelles diffèrent dans des directions différentes de la valeur chromatique du matériau composite de base de telle sorte qu'on obtient une couverture adéquate de la dispersion des valeurs chromatiques des dents naturelles.

2. Ensemble de matériaux composites selon la revendication 1, caractérisé en ce qu'il contient le matériau composite de base en deux variantes ayant la même valeur chromatique mais une opacité différente, une opacité de préférence d'environ 40 % et 60 %, la variante ayant l'opacité inférieure étant conçue pour être appliquée sous la forme d'une couche sur l'un quelconque desdits matériaux composites supplémentaires.

3. Ensemble de matériaux composites selon la revendication 2, caractérisé en ce que lesdits matériaux composites supplémentaires ont sensiblement la même opacité que la variante la plus opaque du matériau composite de base.

4. Ensemble de matériaux composites selon l'une quelconque des revendications 1 à 3, caractérisé en ce qu'un premier desdits matériaux composites supplémentaires, exprimé dans le système NC, est plus blanc et qu'un second est plus noir que ledit matériau composite de base.

5. Ensemble de matériaux composites selon l'une quelconque des revendications 1 à 4, caractérisé en ce qu'un troisième desdits matériaux composites supplémentaires, exprimé dans le système NC, est plus coloré et plus noir que le matériau composite de base.

6. Ensemble de matériaux composites selon l'une quelconque des revendications 1 à 4, caractérisé en ce qu'un troisième desdits matériaux composites supplémentaires, exprimé dans le système NC, est plus jaunâtre et qu'un quatrième est plus coloré et plus noir que le matériau composite de base.

7. Ensemble de matériaux composites selon la revendication 6, caractérisé en ce que le matériau composite de base a la valeur chromatique qui, dans le système NC, est désignée par 1525-Y30R et en ce que lesdits matériaux composites supplémentaires ont des valeurs chromatiques qui, dans le système NC, sont désignées par 1010-Y30R (plus blanc), 3020-Y30R (plus noir), 1030-Y10R (plus jaunâtre) et 3030-Y30R (plus coloré et plus noir).

8. Ensemble de matériaux composites selon l'une quelconque des revendications 1 à 7, caractérisé en ce qu'il comprend également au moins un matériau opaque destiné à couvrir les décolorations sous l'obturation en matériaux composites et un matériau translucide destiné à former une couche superficielle.

9. Procédé de détermination de la couleur d'une obturation dentaire en matériaux composites pour assortir la couleur de l'obturation à celle de l'émail des dents adjacentes, caractérisé en ce que la couleur est déterminée par la sélection d'un matériau composite parmi au moins quatre et de préférene cinq, parmi lesquels un matériau composite de base a une valeur chromatique tridimensionnelle, exprimée en teinte, degré de teinte et noirceur/blancheur, qui correspond à celle d'une sélection moyenne des dents naturelles de telle sorte que l'impression visuelle de la dent et de l'obturation sera la même et les matériaux composites supplémentaires ont des valeurs chromatiques différant de la valeur chromatique du matériau composite de base en étant respectivement plus blanc, plus noir et plus coloré avec une noirceur supérieure ou, dans le cas préféré, plus jaune et plus coloré avec une noirceur supérieure.

10. Procédé selon la revendication 9, caractérisé en ce que la couleur est assortie par sélection de l'épaisseur d'une couche externe d'obturation, le matériau composite de la couche interne étant sélectionné parmi les matériaux composites supplémentaires et la couche externe étant formée par le matériau composite de base qui, dans ce cas, a une opacité inférieure à celle des matériaux composites résiduels, et/ou par un matériau composite transparent.

*Fig.1*

2

1

*Fig.2*

1

1

2

3

4

5

6